# EUROPEAN PATENT APPLICATION

(11) **EP 1 176 206 A2**
(43) Date of publication of application: **30.01.2002**
(21) Application number: 01115677.5
(22) Date of filing: 04.07.2001
(51) Int. Cl.: C12N 15/82, C12N 15/61, C12N 15/29, C12N 9/90, A01H 5/00

(54) **Barley DEAD box protein HVD1 gene induced by salt stress**

(30) Priority: 28.07.2000 JP 2000228941
(71) Applicant: KYUSHU UNIVERSITY, Fukuoka City Fukuoka Pref. (JP)
(72) Inventor: Iba, Koh, Higashi-ku, Fukuoka City, Fukuoka Pref. (JP); Takabe, Tetsuko, Nagoya-City, Aichi Pref. (JP)
(74) Representative: Wibbelmann, Jobst, Dr., Dipl.-Chem.

(57) **Abstract**

A novel gene induced by salt stress, barley HVD1 gene, was provided according to this invention and sequence of the gene was determined. Said gene of this invention revealed to encode RNA helicase. Furthermore, it is expected that resistance to salt stress would be rendered to a plant by incorporating said gene, through stabilizing conformation of RNA.

## Description

### BACKGROUND OF THE INVENTION

### 1. Field of the invention

This invention relates to barley HVD1 gene, a novel gene induced by salt stress, and protein encoded by said gene.

### 2. Description of the Related Art

Food crisis is expected to occur in the near future, caused by increase of population and alteration of environment. Therefore, in order to cope with the problem, there is a great demand on development of an agricultural technique that enables efficient production of crops. The amount of agricultural production tends to be affected by various environmental stress. It deserves to be a serious problem in the field of agriculture. Therefore, production of a plant, exhibiting resistance to various environmental stress, is needed.

In response to environmental stress, some physiological or metabolic alteration occurs in a plant. That is, increase or decrease in expression of various proteins occurs in the course of such response. Alteration of genetic expression caused by environmental stress is one factor that is contributing to control of protein component of a plant. Analysis of a gene, expressing under some environmental stress, is important to understand the mechanism involving stress resistance.

In the past investigations, some genes, induced by osmotic-pressure stress or drying stress, were isolated from various plants (Skriver, K. and Mundy, J. (1990) Plant Cell 2: 503-512) (Bray, E. A. (1993) Plant Physiol. 103: 1035-1040) (Ingram, J. and Bartels, D. (1996) Plant Mol. Biol. 47: 377-403). A family of gene called "lea gene" is included in such genes. The lea genes (late embryogenesis abundant genes) were isolated as a group of genes expressed abundantly in late stage of embryogenesis. It was found that said genes are also expressed in a leaf subjected to moisture stress. LEA proteins are classified into six groups according to their amino acid sequences. It was assumed that the function of the proteins was related to isolation of an ion, protection of protein or cell membrane or retaining of protein structure like a chaperon.

A gene, involved in regulation of osmotic stress, was also isolated (Bohnert, H. J., et al. (1995) Plant Cell 7:1099-1111). Concerning the gene, functions, such as synthesis of compatible solute, sequestration and excretion of ion or function as water channel, have been presented. In addition, following biochemical factors have been isolated. These are proteolytic factors such as protease or ubiquitin, factors that compete to proteolysis such as chaperon or protease inhibitor, protein kinase or nucleoprotein involved in signal transduction, RNA-binding protein and transcriptional factor. Moreover, many genes, with its function unknown, were isolated and functional analysis on these genes is in progress.

On the other hand, concerning production of a plant with improved resistance to salt stress, methods using suitable solute, such as proline or glycine betaine, have been investigated. However, no method, utilizing RNA stability under salt stress, has been developed so far.

### SUMMARY OF THE INVENTION

Therefore, the inventors have incorporated a gene, which is induced under salt stress condition and participates to conformational stability of RNA. Improved resistance to salt stress might be rendered to a plant according to the novel mechanism described above. When a plant is exposed to salt stress, salt concentration increases in the cell. Under such condition, conformation of a single strand RNA tends to alter and exhibit secondary structure of double strand. As the result of conformational alteration described above, biosynthesis of protein or decomposition of RNA by nuclease are inhibited, which presents harmful effect to a plant body. Then, it was assumed that salt resistance might be rendered to a plant by improving conformational stability of RNA using RNA helicase. It is the object of this invention to obtain a novel gene which enables improvement of resistance to salt stress through such mechanism. Moreover, another object of this invention is to determine base sequence of the gene.

RNA helicase is a RNA binding protein and alters secondary structure or tertiary structure of RNA by rewinding double strand of the RNA. The activity of RNA molecule can be controlled subtly by forming certain secondary structure, such as stem/loop, or rewinding it conversely, which is regarded to be important function of RNA. The inventors have remarked RNA helicase exhibiting such property and searched for a novel helicase induced by salt stress. As the result, the inventors have obtained barley HVD1 gene, a gene encoding RNA helicase of barley. Said gene was not expressed in roots, but expressed in leaves. Moreover, expression of said gene was induced by salt stress. It was assumed that barley HVD1 gene having such feature would stabilize conformation of RNA. Therefore, it would render resistance to salt stress by repairing conformation of RNA damaged under salt stress.

These and other features and advantages of this invention will become apparent upon a reading of the detailed description and drawings.

### BRIEF EXPLANATION OF THE DRAWINGS

The above and other objects and features of the present invention will be further explained in detail hereinafter from consideration of the following description taken in connection with the accompanying drawings, in which:
Fig. 1 is a schematic view showing the motifs preserved among DEAD box family and their functions;
Fig. 2 is a schematic view showing outline of differential displaying method;
Fig. 3 is a photograph showing detection of salt stress response gene of the barley by differential displaying method;
Fig. 4 is a photograph of Northern blot analysis showing expression of BD1 induced by salt stress;
Fig. 5 is a figure showing total base sequences of HVD1 cDNA and the deduced amino acid sequence;
Fig. 6 is a figure showing hydrophobic-hydrophilic plot of HVD1 protein;
Fig. 7 is a figure showing the comparison of amino acid sequences of some known DEAD box proteins and the deduced amino acid sequence of HVD1;
Fig. 8 is a photograph of Southern blot analysis of HVD1 gene and DEAD box gene detected in barley genome;
Fig. 9 is a photograph of Southern blot analysis of HVD1-like gene detected in spinach, rice and barley.

### DETAILED DESCRIPTION OF THE PREFERRED EMBODIMENTS

The inventors have remarked gene known to encode DEAD box protein, which is an ATP dependent RNA helicase. The gene encoding the DEAD box protein revealed to be induced by salt stress and analysis on the DEAD box protein was performed. DEAD box family is an ATP dependent RNA helicase defined by Linder et al. (Linder and P et al. (1989) Nature 337: 121-122). Linder et al. discovered that eIF-4A, a translation initiation factor having RNA helicase activity, and some proteins, with their functions unknown, exhibit similarity on their amino acid sequences. Furthermore, it was discovered that some amino acid sequences were highly preserved among numerous organisms from *Escherichia coli*. to human. One of such conserved sequences was a motif consisting of Asp-Glu-Ala-Asp (DEAD by the 1 character notation). Therefore the group of proteins containing said motif were designated to be DEAD box family.

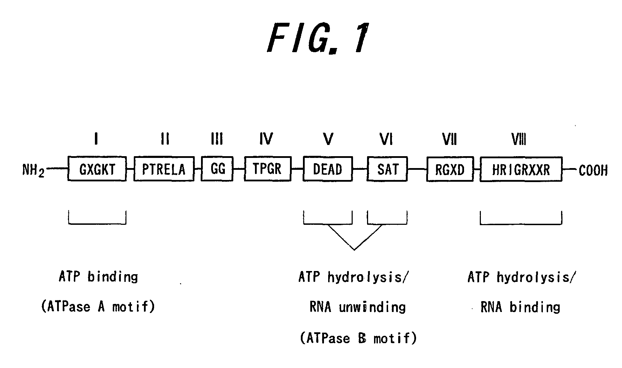

Most of proteins constituting DEAD box family are ATP dependent RNA helicase. As the result of helicase activity of the proteins, modification of secondary structure or tertiary structure of RNA occurs. Since the activity of RNA molecule is known to be controlled skillfully by forming secondary structures, such as stem/loop, or rewinding it conversely, importance of a RNA helicase could be assumed. There are eight consensus sequences (Schmid, S. R. and Linder, P (1992) Mol. Microbiol. 6: 283-292) highly conserved among DEAD box proteins. It is assumed that those sequences are bearing important functions, which are directly relevant to RNA helicase activity, because of the high degree of conservation.
The function of each motif was predicted from function of other proteins having high similarity on the amino acid sequence or identified by investigation on functional failure of mutated proteins produced by site-directed mutagenesis. Fig. 1 shows the motifs conserved among proteins that belongs to DEAD box family and function of those motifs.

DEAD box proteins are involved in various situations, such as cell extension, cell division and formation of reproduction cell. Initiation factor eIF-4A is a member of DEAD box family. Among DEAD box family, eIF-4A has been investigated for a long period and biochemical analysis of the factor, as a RNA helicase, is significantly advanced. The eIF-4A is a subunit of the protein complex (eIF-4F), the complex that recognizes and binds to 5' cap structure of mRNA. The eIF-4A functions to rewind secondary structure of RNA at the position close to 5' end and helps ribosome to bind with mRNA. In addition, DEAD box protein is involved in many stages of genetic expression such as RNA splicing and liposome biosynthesis. Moreover, whenever RNA functions, RNA helicase is involved in it.

Currently, most of DEAD box genes, isolated from plants, are included in initiation factor eIF-4A. The eIF-4A cDNA (NeIF-4A) was isolated from tobacco (*Nicotiana plumbaginifolia*) by Owttrim et al. using eIF-4A of yeast as a probe. In addition, existence of a small gene family was recognized (Owttrim, G. W. et al. (1991) Nucl. Acids Res. 19: 5491-5496). Then, the group of genes, consisting the gene family, were isolated. As the result of the investigation, existence of two groups was confirmed and expression manner of the gene family was further analyzed in detail (Owttrim, G. W. et al. (1994) Plant Mol. Biol. 26: 1747-1757).

The eIF-4A was also isolated from rice (Nishi, R., et al. (1993) Biochem. Biophys. Acta 1174: 293-294) and from wheat (Metz, A. M. and Browning, K. S. (1993) Gene 131: 299-300), not only from tobacco. In addition, a pollen specific expressed eIF-4A, was also isolated from tobacco (*Nicotiana tabacum*) (Brander, K. A. and Kuhlemeier, C. (1995) Plant Mol. Biol. 27: 637-649). DEAD box gene other than eIF-4A has been isolated only from tobacco (*Nicotiana sylvestris*). Itadani et al. (1994) have produced primers according to sequence of conserved motif (Itadani, H. et al. (1994) Plant Mol. Biol. 24:249-252). Then PCR was performed using the primers and nine clones were obtained as the result. Among these clones, full length of DB10 was cloned and base sequence of the clone was determined.
The result of the sequence determination revealed high homology with p68, which is a member of DEAD box family derived from human. It is known that p68 is involved in formation of nucleolus where synthesis of rRNA takes place. Therefore, DB10 might bear the same function.

Incidentally, as a method to isolate a gene, which is expressed under a certain specific condition, the procedures, such as identification of protein by 2-dimensional electrophoresis, differential screening and subtraction method, have been adopted conventionally. In addition to these procedures, a novel procedure was developed recently. It was differential display method developed by Liang and Pardee (1992). The method comprising the steps of RT-PCR using primer A, used for anchoring to mRNA of eukaryotic organism at 3' end, and primer B, having short and arbitrary sequence, labeling with radioisotope, separating by sequence gel and analyzing the obtained sequence (Liang, P. and Pardee, A. B. (1992) Science 257: 967-971). In this method, 12 types of 5-T11VN (V=A, G, C; N = A, T, G, C) and arbitrary sequence of 10 mer were prepared as primer A and primer B respectively and RT-PCR was performed using these primers.

As characteristic for differential display method described above, the method enables detection with high sensitivity, compared with conventional methods such as differential screening method and subtraction method. The differential display method can detect transcript of thymidine kinase gene, which exists only about 30 copies per cell. The inventors have improved the differential displaying method on the basis of the original report by Liang and Pardee (1992) described above. And the inventors have isolated novel gene, which exhibits increased expression in response to salt stress, using said improved differential display method (Yoshida, K. T. et al. (1994) Plant Cell Physiol. 35:1003-1009). That is, primer used for PCR was a primer for RAPD detection and 1 type primer was used for 1 reaction. Then, PCR product, amplified by the same primer from both of 5' region and 3' region of mRNA, was analyzed using the primer described above. Moreover, as agarose gel was used for detection instead of sequence gel, the method was advantageous because cloning by the method was easy.

The inventors performed differential display method using 1 type of primer to obtain two clones, which are salt stress response gene of barley (BD1 and BD2). Among these clones, the expression of BD1 was confirmed to increase in response to salt stress and base sequence of BD1 was determined. Moreover, deduced amino acid sequence was obtained from base sequence and it was confirmed that the amino acid sequence exhibited high homology with that of ATP dependent RNA helicase. As protein encoded by said clone contained all of eight motifs conserved among DEAD box family, the protein was assumed to be DEAD box protein of barley and the protein was named HVD1 (Hordeum vulgare DEAD box protein).

A DEAD box gene originated from a plant, which is specifically expressed under stress condition, has not been isolated until now. Since HVD1 gene according to this invention had characteristic that its expression was increased in response to salt stress, said gene was assumed to be a novel type of DEAD box gene different from eIF-4A. Then, the inventors have isolated and analyzed said gene. The HVD1 protein of this invention was RNA helicase and it was assumed that resistance to salt stress can be rendered to a plant by a novel mechanism. That is, by incorporating HVD1 gene into a plant, conformation of RNA was stabilized and RNA activity was regulated to render resistance to salt stress.

This invention relates to HVD1 gene originated from barley, consisting of a base sequence referred to as base numbers from 1 to 2799 shown in SEQ ID NO: 2 in the sequence list. As described above, HVD1 gene is ATP dependent RNA helicase involved in RNA stability. HVD1 gene according to this invention is a gene involved in resistance to salt stress of a plant, through stabilization of RNA conformation and expression of said gene is induced by salt stress.

According to technique of gene recombination, artificial modification can be achieved at a specific site of basic DNA, without alteration or with improvement of basic characteristic of said DNA. Concerning a gene having native sequence provided according to this invention or modified sequence different from said native sequence, it is also possible to perform artificial modification such as insertion, deletion or substitution to obtain gene of equivalent or improved characteristic compared with said native gene. Moreover, a gene with such mutation is also included in the range of this invention. That is, the gene, consisting of a base sequence hybridizes with said base sequence shown in SEQ ID NO: 2 in the sequence list under stringent condition, means a gene in which 20 or less, preferably ten or less, and more preferably five or less bases of the sequence is deleted, substituted or added to the base sequence shown in SEQ ID NO: 2 in the sequence list. Moreover, such gene exhibits homology 70% or more, preferably 80% or more and still preferably 90% or more with the base sequence shown in SEQ ID NO: 2 in the sequence list. In addition, such gene hybridizes with the base sequence shown in the SEQ ID NO: 2 in the sequence list under stringent condition. Such gene is also within the range of this invention so far as it exhibits the characteristic of HVD1 gene, being induced by salt stress.

Furthermore, this invention relates to HVD1 polypeptide originated from barley, consisting of an amino acid sequence referred to as amino acid numbers from 1 to 764 shown in SEQ ID NO: 1 in the sequence list. The polypeptide is encoded by open reading frame portion of the base sequence indicated by SEQ ID NO: 2 in the sequence list. The polypeptide consisting of an amino acid sequence in which a part of said polypeptide referred to as amino acid sequence shown in SEQ ID NO: 1 is deleted, substituted or added with another amino acid sequence means a polypeptide in which 20 or less, preferably ten or less, and more preferably five or less amino acids of the sequence is deleted, substituted or added to the amino acid sequence shown in SEQ ID NO: 1 in the sequence list. Moreover, such polypeptide exhibits homology 70% or more, preferably 80% or more and still preferably 90% or more with the amino acid sequence shown in SEQ ID NO: 1 in the sequence list. Such polypeptide is also within the range of this invention so far as it exhibits characteristic as HVD1 polypeptide, being encoded by a gene induced by salt stress.

A method to transform a plant by incorporating HVD1 gene derived from a barley into a plant, and a transgenic plant produced by incorporation of said HVD1 gene are also within the range of this invention. The HVD1 gene of this invention is a gene induced by salt stress and it is involved in self defense mechanism of a plant. Therefore, resistance to salt stress can be rendered to a plant by incorporating said gene into a plant. The example of plants, preferred as a target plant, to which said gene induced by salt stress of this invention is incorporated, may include monocotyledonous plants, such as a rice, lily, maize, asparagus and wheat, as well as dicotyledonous plants, such as spinach, carrot, soybean, tomato and potato.

A conventional method known in this art, as a method to produce a transformant, can be utilized. A vector available in this invention may include a plasmid vector, such as pBI121 and pBI221, but not limited to them. Such vector can be incorporated into an *Agrobacterium* strain, then a callus or a plantlet can be transfected by the *Agrobacterium* strain to produce a transgenic plant. Furthermore, a seed from such transgenic plant can be obtained. The method to incorporate said plant gene of this invention is not limited to Agrobacterium method and other methods, such as particle gun method and electroporation method, can be also utilized for incorporation of the gene.

### DETAILED DESCRIPTION OF EMBODIMENTS

### (Plant material)

In this experiment, barley (*Hordeum vuglare* L. cv. Harunanijyo) was used as plant material. Moreover, salt stress treatment was started by addition of 50 mM NaCI to hydroponics medium, which was performed one month after seedling. Concentration of NaCI was increased to 100 mM at the second day of salt stress treatment. Thereafter, concentration of salt was increased in stepwise manner in increments of 100 mM every two days, until finally reaching to 300 mM concentration. The green leaf and the root of barley were harvested at the second day of cultivation in hydroponics medium containing 300 mM of NaCl and the harvested samples were used for subsequent experiment.

### (Strain)

*Escherichia coli* strain DH5α was used for transformation at production of plasmid and *Escherichia coli* strain XL1-Blue MRF' SOIR was used for preparation of cDNA library, respectively. The *Escherichia coli* strains were cultured at 30 to 37°C in culture medium.

### (Enzyme, reagents and the others)

Preparations of total RNA from plant cell, Northern blot analysis, cloning of PCR product, determination of base sequence, labeling of probe DNA, preparation of genomic DNA of plant and Southern blot analysis were performed according to conventional methods.

### (Preparation of poly (A)⁺RNA)

Preparation of poly (A)⁺RNA from total RNA of green leaf, derived from barley with or without exposure to salt stress, was performed using oligo (dT) column. Solution of total DNA (7mg: 4mg/ml) was diluted to 1/2 concentration using 2X binding buffer (1 M NaCl, 10 mM Tris-HCl pH 7.5, 5 mM EDTA, 0.5% SDS). The RNA solution was heated at 70°C for 5 minutes, then cooled on ice. Oligo (dT) column (300 mg of oligo (dT) cellulose [Pharmacia Biotech] was used) was washed by binding buffer (0.5 M NaCl, 10 mM Tris-HCl pH 7.5, 5 mM EDTA, 0.5% SDS) and RNA solution described above was applied to the column, subsequently eluted at flow rate of 10 ml/h. After twice washing of the column using 5-fold volume of binding buffer, RNA was extracted using extraction buffer (10 mM Tris-HCl pH 7.5, 5 mM EDTA), then the extracted RNA was collected. RNA was precipitated by adding 1/10 amount of 3 M sodium acetate and 2-fold volume of ethanol, then the mixture was left stand at -20°C over night for precipitation. It was centrifuged at 15,000 rpm for 30 minutes, pellet obtained by the centrifugation was washed with 70% ethanol and the pellet was dried. It was dissolved in 50 µl of sterilized distilled water, then poly (A)⁺RNA was obtained. Under this condition, about 1 % of total RNA was recovered.

### (Differential display: Synthesis of first strand cDNA)

First strand cDNA was synthesized from poly (A)⁺RNA by reverse transcription. First, 1 nmol random hexamer was added to 5 µg poly (A)⁺RNA and the volume was adjusted to 50 µl with sterilization water. The solution was left 70°C for 10 minutes, subsequently cooled on ice. A hundred µl of reaction mixture for reverse transcription reaction (1X reverse transcription buffer [appended to enzyme], 500 µM of dNTP mix, 10 µM of random hexamer, 10 mM of DTT, 100 µl of 10 U/µl SuperScript IITM RNase H Reverse Transcriptase [GIBCO BRL]) was prepared by adding reagents and enzyme to the poly (A)⁺RNA solution, then it was reacted at 37°C for 1 hour. According to this condition, 600 ng of first strand cDNA, corresponding to about 10% of poly (A)⁺RNA, was obtained.

### (PCR)

PCR and analysis of PCR product were performed according to the procedure of Yoshida et al. (Yoshida K, et al. (1994) Plant Cell Physiol. 35:1003-1009). A primer consisting of 12 bases containing one specific sequence (primer for RAPD detection) was used for PCR. The sequence of the primer, composition of reaction solution and reaction condition are as follows. 5'-ATCAGCGCACCA-3' (Common's primer, A(X): BEX) Composition of reaction mixture : 1 ng of first strand cDNA, 1X PCR buffer solution (appended to enzyme), 200 µM of dNTP mix, 1 µM of primer and 0.025 U/µl AmpliTaq DNA polymerase (PERKIN ELMER)
Reaction condition : 95°C [2 minutes] (x1), 95°C [1 minute], 35°C [1 minute], 72°C [2 minutes] (x40), 72°C [5 minutes] (x1)
Here, PCR was performed using GeneAmp PCR System 9600 (PERKIN ELMER).

### (Comparison of PCR product)

Solution obtained by PCR was fractionated by 1.5% agarose gel electrophoresis and DNA was detected by ethidium bromide staining. PCR product, exhibiting difference by presence or absence of exposure to salt stress, was isolated and purified by low melting point electrophoresis using 1.5% agarose gel.

### (Homology search of a base sequence and an amino acid sequence)

Homology search of the base sequence and the amino acid sequence was performed by E-mail on databases, such as Genebank and EM BL, using blast program.

### (Preparation of cDNA library)

cDNA library was prepared according to the procedure of Gubler and Hoffman (Gubler U. and Hoffman B.J. (1983) Gene 25: 263). First strand cDNA was synthesized by the procedure described above. Then second strand was synthesized by adding enzyme and reagents (92.3 µl of sterilization distilled water, 32 µl of 5X second strand synthesis buffer solution containing : [94 mM Tris-HCl pH9.6, 453 mM KCl, 23 mM MgCl₂, 750 µM β- NAD, 50 mM (NH₄)₂SO₄], 3 µl 10 mM dNTP mix, 6 µl 0.1M DTT, 2 µl DNA ligase [7.5 U/µl], 4 µl DNA polymerase [10 U/µl], 0.7 µl RNaseH [2 U/µl]) to reaction solution (20 µl) in the order. Then the resulting reaction solution was further incubated for 5 minutes at 16°C. T4 DNA polymerase was added to make 10 units per 1 µg of the first strand, then the resulting reaction solution was further incubated for 5 minutes at 16°C.
The reaction stopped by addition of 10 µl of 0.5 M EDTA, 1/10 amount of 3 M sodium acetate and twice volume of ethanol were added to the reaction solution, then it was left stand at -20°C overnight.

The solution was centrifuged for 30 minutes at 15,000 rpm. The obtained pellet was washed with 70% ethanol and the pellet was dried. It was dissolved in 20 µl of TE buffer solution. To this solution, 2.5 µl of EcoRI-NotI-BamHI adapter (100 pmol/µL, TAKARA SHUZO Co.), A liquid (80 µl) and B liquid (10 µl) of DNA ligation kit (TAKARA SHUZO Co.) were added and ligation reaction was performed at 16°C for 4 hours. The pellet obtained by ethanol precipitation was dissolved into 20 µl of TE buffer and it was loaded onto Sepharose CL-4B packed column. It was eluted using extraction buffer (TE, pH 7.6, 0.1 M NaCI) and size fractionation of cDNA was performed. To perform phosphorylation treatment of the adapter, 80 µl 10X L/K buffer (50 mM Tris-HCl pH 8.0, 10 mM MgCl₂, 5 mM DTT, 100 µM ATP) and 8 µl T4 polynucleotide kinase (10 units/µl) were added to the collected fraction (800 µl), mixed gently and subsequently incubated at 37°C for 1 hour.

The reaction mixture was extracted with phenol/chloroform, precipitated using ethanol and the resulting pellet was dissolved into 10 µl of ligation solution A. One µl of Lambda phage vector Lambda ZAP II (Predigested Lambda II/EcoRI cloning kit: Stratagene) digested by EcoRI and 10 µl of ligation solution B were added to above solution and mixed sufficiently. Then ligation reaction was performed overnight at 16°C. The reaction mixture was precipitated by ethanol, the resulting pellet was dissolved into 5 µl of TE buffer.

### (Subcloning to vector)

DNA cloned to lambda phage vector Lambda ZAP II was abscissed and conjugated by infection of helper phage, subsequently cloned to pBluescript SK (In vivo excision).

To 200 µl of phage suspension (>1X10⁵ phage particle), 200 µl of fresh host *Escherichia coli* XLI-Blue MRF' (OD₆₀₀ = 1.0) and 1 µl of ExAssist helper phage were added and mixed, then incubated for 15 minutes at 37°C. The solution was added to 5 ml of 2X YT medium (1.6 % polypeptone, 1% yeast extract, 0.5 % NaCl) and it was cultured with shaking at 37 °C for 3 hours. The culture solution was treated at 70 °C for 20 minutes, centrifuged at 6,000 rpm for 5 minute, then the resulting supernatant was stored at 4°C. A portion (10-20 µl) of supernatant was added to 200 µl of host *Escherichia coli* SOIR (OD₆₀₀ = 1.0) and it was cultured with shaking at 37°C for 15 minutes. The culture solution was spread on LB/Amp plate (1.0% polypeptone, 0.5 % yeast extract, 1.0 % NaCl, 100 µg/ml ampicillin sodium) uniformly and cultured at 37 °C overnight to obtain colonies.

### (Detection of a gene induced by salt stress)

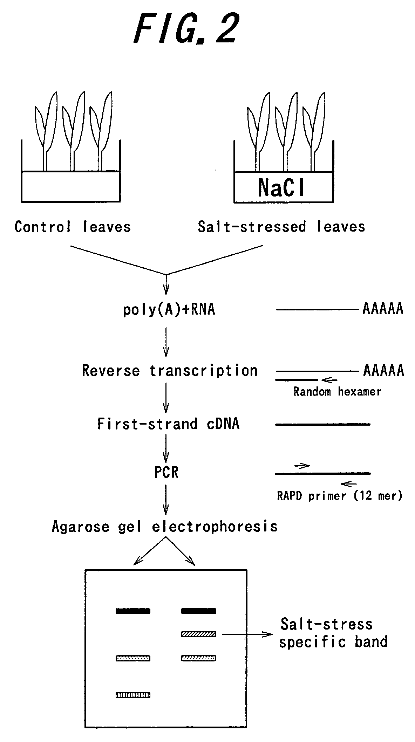

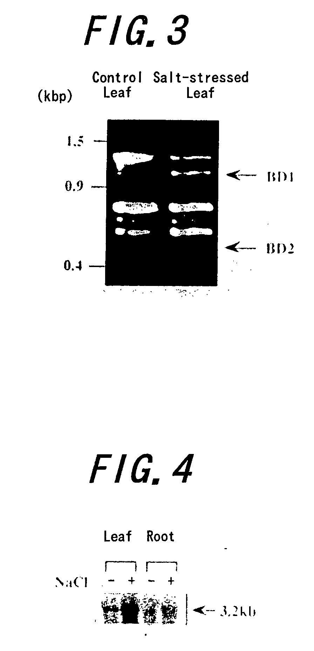

For molecular level investigation on mechanism of salt stress resistance, a gene, exhibiting increased expression under salt stress condition, was isolated and analyzed. The differential display method was used for detection of a gene capable of responding to salt stress. The scheme of the differential display method used in this invention is shown in Fig. 2. Poly (A)⁺RNA was prepared from each of barley leaf with or without exposure to salt stress respectively, then first strand cDNA was synthesized by reverse transcription. PCR was performed, using this first strand cDNA as a template. As a result of PCR using one primer for RAPD detection, two PCR products, exhibiting increased amplification in barely with exposure to salt stress, were detected. Fig. 3 shows the result of detection of barely gene responding to salt stress, using differential display method. In Fig. 3, the result of control leaf is shown in the right and that of leaf exposed to salt stress was shown in the left. Besides, arrows indicate PCR products (about 1.1 bp and about 0.5 bp) exhibiting increased amounts of amplification in leaf with exposure to salt stress, as the result of PCR.

### (Expression analysis of PCR product)

Northern blot analysis was performed using PCR products of about 1.1 kbp (BD1) or that of about 0.5 kbp (BD2) as a probe, respectively. Using BD1 as a probe, mRNA of about 3.2 kb was detected in the leaf. The result of Northern blot, on expression induction of BD1 by salt stress, is shown in Fig. 4. In Fig. 4, expression of the gene was investigated using total RNA derived from leaf (Leaf) and root (Root) of barley, with (NaCl+) and without (NaCl-) exposure to salt stress. Concerning leaf, expression of the gene was detected even on leaf without exposure to salt stress and expression of the gene increased about 8 times by exposure to salt stress. On the one hand, the amount of expression of the gene detected on root was lower compared with that of leaf. Nonetheless, expression of the gene was also detected without exposure to salt stress, expression of the gene increased about 3 times by exposure to salt stress. Concerning BD2, it was not detected by Northern blot analysis, probably because the amount expression of BD2 was very low. Therefore, as expression of BD1 was confirmed to be increased by salt stress, BD1 was cloned and it was used for further analysis.

### (Identification of PCR product)

Then base sequence of cloned BD1 was determined. Assumed amino acid sequence, obtained from determined base sequence, was analyzed on database. As the result, the amino acid sequence exhibited high homology with ATP dependent RNA helicase. The group of ATP dependent RNA helicases constitute DEAD box family and eight conserved motifs are observed on this family (Figure 1). The PCR product contained seventh motif and the eighth motif of said eight motifs.

### (Cloning of full length cDNA and feature of the sequence)

In order to clone full length cDNA containing BD1, cDNA library was prepared from mRNA derived from barely leaf exposed to salt stress. About 20,000 independent clones were screened using BD1 as a probe and two positive clones were obtained. The two clones are originated from a same gene and one clone, encoding longer base sequence, was further analyzed.

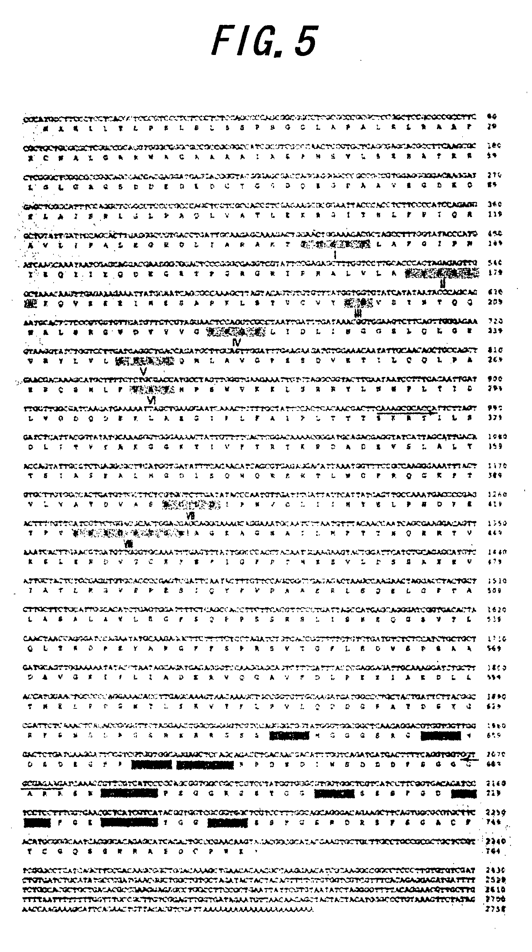

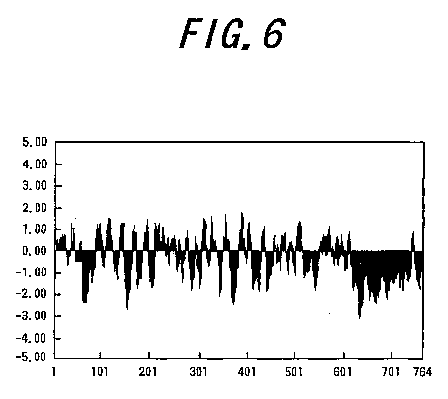

As the result of total sequencing, HVD1 cDNA was reveled to comprising 2,799bp with 22bp of poly A. Total base sequence of HVD1 cDNA and deduced amino acid sequence obtained from the base sequence are shown in Fig. 5. In Fig. 5, the portion indicated by yellow quadrangle, the portion indicated by green quadrangle and the portion indicated by blue quadrangle correspond to motifs (I-VIII) conserved among DEAD box family proteins, RGG motif and RSSS motif, respectively. The arrow shows the sequence recognized by the primer for RAPD detection at performance of differential display. Although sequence similar to poly-A additional signal sequence was recognized, the typical poly-A additional signal (AATAAA) was not recognized. One open reading frame, encoding polypeptide comprising 764 amino acids, exists in cDNA of HVD1. The molecular weight evaluated from this amino acid sequence was 81.8kDa with isoelectric point of 7.67. The result of hydrophobic-hydrophilic plot of HVD1 protein, obtained using parameter value of Kyte & Doolittle, is shown in Fig. 6. As the result of hydrophobic-hydrophilic plot, a hydrophobic region such as transmembrane domain was not recognized. However, a highly hydrophilic region was recognized at its carboxyl terminal region (Fig. 6).

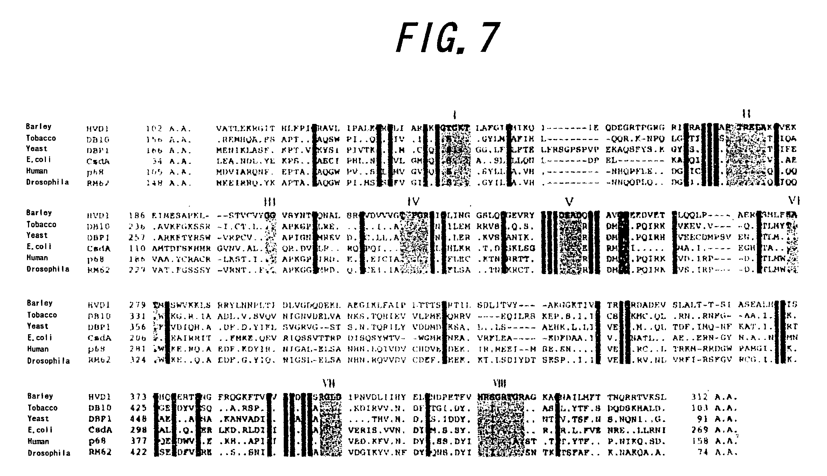

The amino acid sequence of HVD1 and those of known ATP dependent RNA helicases are compared in Fig. 7. In Fig. 7, the portion indicated by yellow quadrangle, the portion indicated by green quadrangle and the portion indicated by blue quadrangle correspond to motifs conserved among DEAD box family proteins, amino acids conserved among DEAD box family proteins and amino acids conserved among six proteins, respectively. That is, amino acid sequences of HVD1 from barley, DBP1 from yeast, CsdA from Escherichia coli, p68 from human, and RM62 from drosophila were compared and the result was shown in Fig. 7. HVD1 contained all eight motifs conserved among DEAD box family proteins. Although the region containing eight motifs exhibited high homology among DEAD box proteins, a protein exhibiting high homology at amino terminal or carboxyl terminal portion with HVD1 have not cloned yet. Moreover, HVD1 contained the longest carboxyl terminal, compared with ATP dependent RNA helicase cloned until now. RGG sequence was repeated 5 times in the hydrophilic region described above.
This motif is RNA binding motif observed among RNA binding proteins. Furthermore, RSSS sequence was repeated 4 times in this region, although its function was not known.

### (Feature of HVD1)

In the region containing eight motifs, some amino acids are preserved in HVD1, except for the consensus sequence. However, a ATP dependent RNA helicase, having high homology in this region, has not isolated until now. Similarly, a protein, having homology at amino terminal region or carboxyl terminal region, was not known yet. Especially HVD1 contained long carboxyl terminal region and it was revealed to be the longest in DEAD box protein isolated until now. This carboxyl terminal region exhibited high hydrophilicity and RGG motif existed in the region. It was considered that the carboxyl terminal of HVD1 was non-specific RNA binding region, since RGG motif was one of the RNA binding motifs identified in RNA binding proteins.

Among DEAD box proteins isolated from human, rat, drosophila and yeast, some proteins are known to contain RGG motif. Among them, Ste13 protein of fission yeast (Maekawa H. et al. (1994) Mol. Gen. Genet. 244:456-464) contained RGG motif at its carboxyl terminal region, like HVD1. Ste13 gene was isolated from Ste13 mutant exhibiting failure of conjugation and meiosis under nitrogen-source starvation condition. Therefore, its function might be involved in expression control of a gene after transfer of the gene. When RGG motif was deleted from Ste13 protein (Shimoda, C. et al. (1994) The Second UK-Japan Cell Cycle Workshop), significant decrease of RNA binding ability was observed. Therefore, it was assumed that the carboxyl terminal region was RNA binding region.

In the region assumed to be RNA binding region of HVD1, a motif containing 4 times repeated RSSS sequence was observed. Though the function of RSSS sequence was not known, the motif might possibility involved in specificity of RNA molecule. As RNA binding ability observed on the consensus sequence of DEAD box protein was low, support of other RNA binding domains, such as HVD1 and Ste13, might be necessary for stable binding with RNA molecule. The proteins, containing RNA binding region other than the consensus sequence, exhibited RNA helicase activity by themselves. However, proteins like elF-4A had deficiency on RNA binding region. Such proteins exhibited its activity as a RNA helicase, only when said proteins formed complex with other proteins containing RNA binding domain.

### (Copy number of HVD1 in the genome)

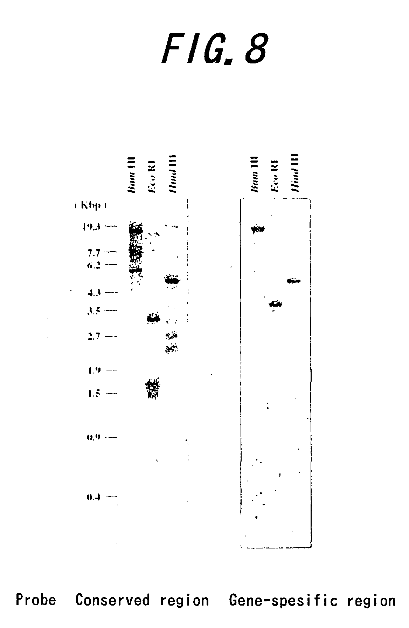

Copy number of HVD1 gene in barley genome was identified by southern plot analysis. The result of detection of HVD1 gene and DEAD box gene, performed on barley genome is shown in Fig. 8. That is, copy numbers of the genes were analyzed on the genome using HVD1 gene specific region as a probe. Then the region conserved in DEAD box gene of HVD1 was used as a probe to detect genes that belong to DEAD box family. As the result of analysis on barley genome, using gene specific region in the 3' side of HVD1 cDNA as a probe (Figure 8, right), only one band was detected and therefore it was revealed that 1 copy per one haploid of HVD1 gene existed in the genome. However, when the conserved region comprising eight motifs of HVD1 cDNA was used as a probe (left-hand side in Figure 8), plural bands were detected. The result indicated existence of DEAD box gene family in barley.

### (HVD1 gene in other plants)

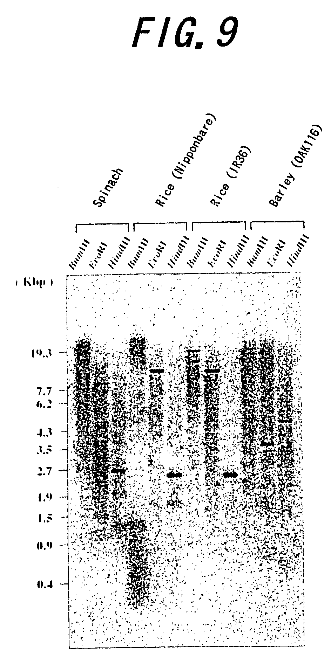

Existence of HVD1 like gene, in a plant other than barely, was further analyzed. The result of detection of HVD1 like gene, analyzed by southern blotting using the genomic DNA of spinach, rice (2 species) and barley, was shown in Fig. 9. As the result of southern blot analysis, using gene specific region of HVD1 cDNA as a probe, signal as a blotting band was detected in dicotyledonous plant such as spinach and monocotyledonous plant such as rice. It is considered that HVD1 like genes exist in spinach and rice plant as well.

### (Function of HVD1)

As expression of HVD1 increased under salt stress, it was assumed that activity of HVD1 would not be inhibited by salt stress. The activity of DEAD box protein isolated from human, named p68, is known to be enhanced by salt (100 mM NaCl). Furthermore, as p68 is significantly resistant to salt stress, it can bind to RNA under existence of salt more than 250 mM. Therefore, it is predicted that HVD1 would also exhibit significant resistance to salt, like p68.

As described above, when salt concentration increased in a plant cell by salt stress, a single strand RNA would tend to form altered secondary conformation, such as single strand or stem/loop (Jacobson, A. B. (1976) Proc. Natl. Acad. Sci. USA 73: 307-311) (Herbeck, R. et al. (1976) Biochem. Biophys. Acta 418: 52-62). By forming secondary conformation described above, some harmful effects, such as inhibition of protein synthesis (Baglioni, C. et al. (1978) Eur J. Biochem. 92:15-163) and inhibition of nuclease degradation (Edy, V G. et al. (1976) Eur J. Biochem. 61: 563-572), would be caused.

DEAD box protein is involved in protein synthesis and elF-4A, which is an initiation factor of translation, is included in DEAD box protein. However, until now, there have been no report describing a DEAD box protein induced by stress. Therefore, HVD1 protein of this invention is the first report of such protein. It is considered that the function of HVD1 is related to prevention of harmful effects caused by salt stress such as inhibition of protein synthesis or degradation of RNA decomposition. Otherwise, the function of HVD1 would contribute to RNA stability under salt stress. HVD1 gene of this invention is involved in resistance to salt stress according to a novel mechanism, which have been not recognized until now. Therefore many applications can be achieved using HVD1 gene.

A novel gene induced by salt stress, barley HVD1 gene, was provided according to this invention. Said gene of this invention revealed to encode RNA helicase. Furthermore, it is expected that resistance to salt stress would be rendered to a plant by incorporating said gene, through stabilizing conformation of RNA.

## Claims

1. A polypeptide consisting of an amino acid sequence of following (a) or (b):
(a) an amino acid sequence referred to as amino acid numbers from 1 to 764 shown in SEQ ID NO: 1 in the sequence list,
(b) an amino acid sequence in which a part of said amino acid sequence (a) is deleted or another amino acid sequence is added to said amino acid sequence (a) or a part of amino acid sequence (a) is substituted with another amino acid sequence, the amino acid sequence (b) being encoded by a gene induced by salt stress in barely.

2. A polypeptide consisting of an amino acid sequence exhibiting at least 70% of homology with an amino acid sequence referred to as amino acid numbers from 1 to 764 shown in SEQ ID NO: 1 in the sequence list.

3. A gene encoding the polypeptide according to claim 1.

4. A gene encoding the polypeptide according to claim 2.

5. A gene consisting of a base sequence of following (c), (d) or (e):
(c) a base sequence referred to as base numbers from 1 to 2,799 shown in SEQ ID NO: 2 in the sequence list,
(d) a base sequence in which a part of base sequence (c) is deleted or another base sequence is added to said base sequence (c) or a part of base sequence (c) is substituted with another base sequence, the base sequence (d) being induced by salt stress in barely, or
(e) a base sequence that hybridizes with said base sequence (c) under stringent condition, the base sequence (e) being induced by salt stress in barely.

6. A gene consisting of a base sequence exhibiting at least 70% of homology with a base sequence referred to as base numbers from 1 to 2,799 shown in SEQ ID NO: 2 in the sequence list.

7. A method to render resistance to salt stress to a plant, the method comprising incorporating the gene into said plant according to claim 3.

8. A method to render resistance to salt stress to a plant, the method comprising incorporating the gene into said plant according to claim 4.

9. A method to render resistance to salt stress to a plant, the method comprising incorporating the gene into said plant according to claim 5.

10. A method to render resistance to salt stress to a plant, the method comprising incorporating the gene into said plant according to claim 6.

11. A transgenic plant exhibiting resistance to salt stress, produced by incorporation of the gene according to claim 3 into a plant.

12. A transgenic plant exhibiting resistance to salt stress, produced by incorporation of the gene according to claim 4 into a plant.

13. A transgenic plant exhibiting resistance to salt stress produced by incorporation of the gene according to claim 5 into a plant.

14. A transgenic plant exhibiting resistance to salt stress, produced by incorporation of the gene according to claim 6 into a plant.


Sequence listing
<110> President of Kyushu University
<120> HVD1 gene induced by salt stress
<160> 2
<210> 1
<211> 764
<212> Amino acid
<213> Hordeum vulgare L. cv. Harunanijyo
<400> 1
MASLLTLPSL SLSSPSGGLA PALRLRAAFR CWALGRRWAG AAAAIASPNS VLSEHAFKRL 60
GLGAGSDDED EDGYGSDQEG PAAVEGDKDE LAISRLGLPA QLVATLEKRG ITHLFPIQRA 120
VLIPALEGRD LIARAKTGTG KTLAFGIPMI KQIIEQDEGR TPGRGRIPRA LVLAPTRELA 180
KQVEKEIMES APKLSTVCVY GGVSYNTQQN ALSRGVDVVV GTPGRLIDLI NGGSLQLGEV 240
RYLVLDEADQ MLAVGFEEDV ETILQQLPAE RQSMLFSATM PSWVKKLSRR YLNNPLTIDL 300
VGDQDEKLAE GIKLFAIPLT TTSKRTILSD LITVYAKGGK TIVFTRTKRD ADEVSLALTT 360
SIASEALHGD ISQHQRERTL NGFRQGKFTV LVATDVASRG LDIPNVDLII HYELPNDPET 420
FVHRSGRTGR AGKAGNAILM FTTNQRRTVK SLERDVGCKF EFIGPPTMEE VLDSSAEHVI 480
ATLRGVHPES IQYFVPAAER LSQELGPTAL ASALAHLSGF SQPPSSRSLI SHEQGSVTLQ 540
LTRDPEYARG FFSPRSVTGF LSDVSPSAAD AVGKIYLIAD ERVQGAVFDL PEEIAKDLLT 600
MELPPGNTLS KVTKLPVLQD DGPATDSYGR FSNSDRGSRN RRGSSRGGMG GGSRGRGGWD 660
SDEGFRRGGR SSSRPDNDIW SDDDFSGGGA RRSNRSSSPS GGRSSYGGRG GSSSFGDRSS 720
SFGERSSSYG GRGGSSFGSR DRSFSGACFT CGQSGHRASD CPNK 764

<210> 2
<211> 2799
<212> Nucleic acid
<213> Hordeum vulgare L. cv. Harunanijyo
<400> 2
CGCCCGGGCA GGTATTTCTT ATCGCTTCCC CCCTTCCTCC CCCCATGGCT TCCCTCCTCA 60
CGCTCCCGTC CCTCTCCCTC TCCAGCCCCA GCGGCGGCCT CGCGCCCGCG CTCCGGCTCC 120
GCGCCGCCTT CCGCTGCTGG GCGCTCGGCC GCAGGTGGGC GGGCGCCGCC GCGGCCATCG 180
CGTCGCCCAA CTCCGTGCTC AGCGAGCACG CCTTCAAGCG CCTCGGGCTC GGCGCCGGCA 240
GCGACGACGA GGATGAGGAC GGGTACGGGA GCGACCAGGA GGGGCCCGCC GCCGTGGAGG 300
GGGACAAGGA TGAGCTCGCC ATTTCCAGGC TCGGCCTCCC CGCCCAGCTC GTCGCCACCC 360
TCGAGAAGCG CGGAATTACC CACCTCTTCC CCATCCAGAG GGCTGTATTG ATTCCAGCAC 420
TTGAGGGCCG TGACCTGATT GCAAGAGCAA AGACTGGAAC TGGAAAGACG CTAGCCTTTG 480
GTATACCCAT GATCAAGCAA ATAATCGAGC AGGACGAAGG GCGGACTCCC GGGCGAGGTC 540
GTATTCCGAG AGCTTTGGTC CTTGCACCCA CTAGAGAGTT GGCTAAACAA GTTGAGAAAG 600
AAATTATGGA ATCAGCGCCA AAGCTTAGTA CAGTGTGTGT TTATGGTGGT GTATCATATA 660
ATACCCAGCA GAATGCACTC TCCCGTGGTG TTGATGTTGT CGTAGGAACT CCAGGTCGCC 720
TAATTGATTT GATAAACGGT GGAAGTCTTC AGTTGGGAGA AGTAAGGTAT CTGGTCCTTG 780
ATGAGGCTGA CCAGATGCTT GCAGTTGGAT TTGAAGAAGA TGTGGAAACA ATATTGCAAC 840
AGCTGCCAGC TGAACGACAA AGCATGCTTT TCTCTGCGAC CATGCCTAGT TGGGTGAAGA 900
AATTGTCTAG GCGGTACTTG AATAATCCTT TGACAATTGA TTTGGTTGGC GATCAAGATG 960
AAAAATTAGC TGAAGGAATC AAACTCTTTG CTATTCCACT CACAACGACT TCAAAGCGCA 1020
CCATTCTTAG TGATCTCATT ACGGTATATG CAAAGGGTGG GAAAACTATT GTTTTCACTC 1080
GGACAAAACG GGATGCAGAC GAGGTATCAT TAGCATTGAC AACCAGTATT GCGTCTGAGG 1140
CGCTTCATGG TGATATTTCA CAACATCAGC GTGAGAGGAC ATTAAATGGT TTCCGCCAAG 1200
GGAAATTTAC TGTGCTTGTG GCCACTGATG TTGCTTCTCG TGGTCTTGAT ATACCCAATG 1260
TTGATTTGAT TATTCATTAT GAGTTGCCAA ATGACCCCGA GACTTTTGTT CATCGTTCTG 1320
GACGCACTGG ACGAGCAGGG AAAGCAGGAA ATGCAATCTT AATGTTTACA ACCAATCAGC 1380
GAAGGACAGT TAAATCACTT GAACGTGATG TTGGGTGCAA ATTTGAGTTT ATTGGCCCAC 1440
CTACAATGGA AGAAGTACTG GATTCATCTG CAGAGCATGT CATTGCTACT CTGCGAGGTG 1500
TGCACCCCGA GTCGATTCAA TACTTTGTTC CAGCGGCTGA GAGACTAAGC CAAGAACTAG 1560
GACCTACTGC TCTTGCTTCT GCATTGGCAC ATCTGAGTGG ATTTTCTCAG CCACCTTCTT 1620
CACGTTCCCT GATTAGCCAT GAGCAGGGAT CGGTGACACT ACAACTAACC AGGGATCCAG 1680

AATATGCAAG AGGCTTCTTT TCTCCTAGAT CTGTCACCGG TTTTCTGTCT GATGTCTCTC 1740

CATCTGCTGC TGATGCAGTT GGAAAAATAT ACCTAATAGC AGATGAGAGG GTCCAAGGAG 1800

CAGTCTTTGA TTTACCCGAG GAGATTGCAA AGGATCTGCT TACCATGGAA CTGCCCCCAG 1860

GAAACACCTT GAGCAAAGTA ACAAAGCTGC CGGTGTTGCA AGATGATGGC CCTGCTACTG 1920

ATTCTTACGG CCGATTCTCA AACTCAGACC GGGGTTCTAG GAACCGGCGG GGGTCGTCCA 1980

GGGGCGGTAT GGGTGGCGGC TCAAGAGGAC GTGGTGGTTG GGACTCTGAT GAAGGATTCC 2040

GTCGTGGTGG CAGGAGCTCC AGCAGACCTG ACAACGACAT TTGGTCAGAT GATGACTTTT 2100

CAGGTGGTGG TGCGAGAAGA TCAAACCGTT CGTCATCCCC CAGCGGTGGC CGCTCGTCCT 2160

ATGGTGGGCG TGGTGGCTCG TCATCCTTCG GTGACAGATC CTCCTCCTTT GGTGAACGCT 2220

CATCGTCATA CGGTGGTCGC GGTGGCTCGT CCTTTGGCAG CAGGGACAGA AGCTTCAGTG 2280

GCGCGTGCTT CACATGCGGG CAATCAGGGC ACAGAGCATC AGACTGCCCG AACAAGTAGA 2340

CGGCGCATAC GAAGTGCTGC TTGCCTGCCG CGCTGCTCCG TTCGGGCCTC ATCAGCTTCC 2400

GACAAGCGGC TGGACAAAGC TGAACACAAC GCCAAGGAAC ATCGCAAGGC CGGCCTCCCT 2460

TGTGTGTCGA TCTGTGATCT GCATATGCCC GATGAACGGC TGGCTGTGCT AGATACTACT 2520

ACAGTTTTGT GTGGTCGTCG TTTCACAGAG GAGATGATTT TTCTGGCACG CTGCTGACAC 2580

GCCGAAGGAG AGCCTGGCCT TCCGCTGAAT TATTCGTGTA ATATCTAGGG GTTTTACAGG 2640

AACGTTGCTT GTTTTAATTT TTTTTGGTTT GCCGCTTGTC GGAGTTGGTG ATAGAATGTT 2700

AACAACAGCT ACTACTACAT GGGCCCTGTA AAGTTCTATA GAACCAAGAA AGCATTCAGA 2760

ACTGTTACAC GTCGATTAAA AAAAAAAAAA AAAAAAAAA 2799